# EUROPÄISCHE PATENTSCHRIFT

(11) **EP 2 641 583 B1**
(45) Veröffentlichungstag und Bekanntmachung des Hinweises auf die Patenterteilung: **21.10.2015**
(21) Anmeldenummer: 13000745.3
(22) Anmeldetag: 13.02.2013
(51) Int. Cl.: A61K 6/00

(54) **Haftcreme für Zahnprothesen aus natürlichen Bestandteilen**
Adhesive cream made from natural constituents for dental prostheses
Crème adhésive pour prothèses dentaires à base de composants naturels

(30) Priorität: 21.03.2012 EP 12075031
(43) Veröffentlichungstag der Anmeldung: 25.09.2013
(73) Patentinhaber: Kotsokolos, Christos, 16259 Bad Freienwalde (DE)
(72) Erfinder: Kotsokolos, Christos, 16259 Bad Freienwalde (DE)
(74) Vertreter: Castell, Klaus

(56) Entgegenhaltungen:
- WO-A2-2011/106817
- DE-A1-102009 039 363
- GB-A- 967 751
- US-A1- 2007 185 232
- US-A1- 2011 027 328
- US-B2- 6 696 058

## Beschreibung

Die Erfindung betrifft eine Haftcreme zur Fixierung einer Gaumenplatte einer Zahnprothese am menschlichen Gaumen und ein dazu korrespondierendes Verfahren zur Herstellung einer Haftcreme.

Zur Fixierung von Gaumenplatten einer Kieferprothese oder Zahnprothese am menschlichen Gaumen ist es bekannt, Haftcremes einzusetzen, welche zwischen der Schleimhaut des Gaumens und der Gaumenplatte befindlich die Gaumenplatte am menschlichen Gaumen fixieren. Im Mund herrscht ein leicht basisches, aufgrund des Speichels feuchtes Milieu. Eine weitere Umgebungsbedingung ist, dass der menschliche Speichel bereits Verdauungsenzyme aufweist, der im Mund befindliche, körperfremde Substanzen andaut oder zumindest für die weitere Verdauung im Magen-Darm-Trakt des Menschen vorbereitet. Die physikalischchemischen Umgebungsbedingungen im Mund erfordern somit eine sehr sorgfältige Auswahl der Zutaten der Haftcreme, damit die Haftcreme einerseits eine akzeptable Haftkraft entfaltet, andererseits dabei nicht angedaut oder verdünnt wird oder sogar durch Aufnahme von Flüssigkeit aus dem Speichel einer Quellung unterliegt.

Neben den für die Ausbildung einer stabilen Haftcreme herausfordernden Umgebungsbedingungen im Mundbereich, ist es eine Anforderung an eine Haftcreme, dass diese auch keinen Geschmack absondert, der das Tragen einer Kiefer- oder Zahnprothese mit Gaumenplatte unangenehm macht und dem Träger den Genuss von Speisen und Getränken verwehrt.

Schließlich gelangt stets ein geringer Teil der Haftcreme durch Auflösung im Speichel in den menschlichen Verdauungstrakt und kann dort gegebenenfalls durch seine Unverdaulichkeit Beschwerden verursachen.

Es ist bekannt, dass bei der Verwendung von Haftcreme und Haftpulver mit gattungsgemäßen Substanzen aus dem Stand der Technik Entzündungen und Schwellungen der Mundschleimhaut auftreten können, wobei die Entzündungen unter anderem durch Verletzungen der Mundschleimhaut beim Entfernen der Prothese entstehen, wenn diese zum Zweck der Säuberung aus dem Mund entfernt wird. Nicht selten passiert es, dass eine Haftcreme eine so intensive Haftung der Gaumenplatte am menschliche Gaumen zur Verfügung stellt, dass die empfindliche Gaumenschleimhaut beim Lösen der Gaumenplatte traumatisiert wird. Neben den Verdauungsbeschwerden kann die Haftcreme auch mittelbar ursächlich sein für Reizungen im Rachen sowie für Probleme in den Bronchien.

Häufige Inhaltsstoffe von Haftcremes sind Mineralöle, Vaseline, aber auch modifizierte Stärke, wie beispielsweise Carboxymethylcellulose. Die Mineralöle und Mineralfette, wie Vaseline, können eine Übersäuerung im Magen verursachen, was zu einem Singultus und auch zu einer Störung der Verdauung im Zwölffingerdarm führen kann. Viele Menschen, die eine Unverträglichkeit beispielsweise von Gluten zeigen, und dies nicht unbedingt nur bei Zöliakie, weisen in der Regel auch eine Intoleranz gegenüber modifizierten Stärken auf, die in Kombination mit anderen Zuckern zu Verdauungsbeschwerden führen, wie beispielsweise fauligem Stuhl, Durchfall oder Verstopfung.

In der deutschen Offenlegungsschrift DE2133709 wird ein Verfahren zur Herstellung einer Haftcreme für Zahnprothesen offenbart, in welchem ein Polymer aus Polymethylvinylether-maleinsäureanhydrid, das mit einem Alkohol verestert wird, unter Mischung mit Mineralöl und Vaseline als Haftcreme dient. Diese Haftcreme zeigt die eingangs erwähnten Nachteile der Unverdaulichkeit und der Magen-Darm-Reizung bei Empfindlichkeit gegenüber künstlichen Polymeren.

In der deutschen Offenlegungsschrift DE4316115A1 wird ein Haftmittel für Zahnprothesen offenbart, das als hydrophobes Oleogel ein dickflüssiges Paraffinum mit naphtenischer Struktur aufweist. Diese dickflüssigen Paraffine sind Bestandteil von Mineralfetten, welche auch die eingangs erwähnten Nachteile aufweisen.

In der deutschen Patentschrift DE 198 15 547 C1 ist eine Lehre niedergeschrieben, welche als Haftmittel für Zahnprothesen Rübenpektin oder ein davon abgeleitetes Pektinat aufweist. Pektinate können durch Verdauung in verschiedene Zucker und Zuckerester verwandelt werden, die eine Unverträglichkeitsreaktion im Magen-Darm-Trakt auslösen können.

In dem deutschen Gebrauchsmuster DE 295 03 302 wird eine Haftcreme offenbart, welche einen antiphlogistischen Anteil an Kamilleextrakt aufweist, um das unter dem Haftgel zur Entzündung neigende Schleimhautgewebe des Gaumen zu beruhigen und um Entzündungen vorzubeugen. Als weitere, wesentliche Bestandteile werden künstliche Polymere angegeben, welche die eingangs erwähnten Unverträglichkeiten bei dauernder Exposition auslösen können.

Auch in der deutschen Patentschrift DE 102005031771 B4 wird eine Haftcreme auf Carbomer-basis offenbart, wobei das Carbomer als Füllmittel für eine paraffinartige Substanz dient. Auch diese Haftcreme überwindet nicht die eingangs erwähnten Nachteile.

Schließlich wird in der internationalen Patentanmeldeschrift WO 2011/088989 A1 eine Haftcreme offenbart, welche auf Cellulosederivaten, Maleinsäureanhydrid-Copolymer und einem Trihydroxystearin, einer Wachssäure, basiert. Auch diese Rezeptur weist die eingangs erwähnten Nachteile auf.

Hinsichtlich der durch modifizierte Stärke hervorgerufenen Unverträglichkeitsprobleme tritt die Druckschrift GB 967 751 A in den Blick, in der zum Vergleich für das dort vorgeschlagene Haftmittel auf das zeitgenössische Handelsprodukt Poligrip verwiesen wird, das in Ausführungsformen Flohsamenstärke enthalten könne. Keine Hinweise sind für einen solchen Fall jedoch gegeben, in welchen Mengenanteilen Flohsamenstärke vorzusehen ist, ob sie in modifizierter Form verwendet wird und ob sie mit anderen Stärkearten gemischt werden müsste, um den gewünschten Haftungseffekt zu erzielen.

Auch in der Druckschrift US 6 696 058 B2 wird ein wasserhaltiges, enzymatisches Klebemittel für Gebissprothesen offenbart, das in Ausführungsformen Stärke zur Verdickung enthalten könne. Unklar bleibt, welche Stärkesorten im Falle der Verwendung vorteilhafter, nicht modifizierter Stärken zu verwenden sind und in welchem Gewichtsanteil sie dann zu verwenden wären, um einerseits die erwünschte Verträglichkeit und andererseits die notwendigen Haftungseigenschaften zu gewährleisten. Auch in anderen Druckschriften finden sich Ansätze, Stärkesorten pflanzlichen Ursprungs in dentalen Klebemitteln zu verwenden (vgl. z.B. WO 2011/106817 A2, US 2007/185232 A1 und US 2011/027328 A1).

Die Verwendung von zerkleinerten Leinsamen und Flohsamenschalen ist aus der Druckschrift DE 10 2009 039 363 A1 für Verfahren zur Bereitstellung von Nahrungsmittel-Zwischenprodukten und -Endprodukten, die eine gesunde Ernährung gewährleisten sollen, bekannt.

Viele der bei gegebenen Haftmitteln für Zahnprothesen bestehenden Probleme und Unverträglichkeiten gehen auf deren künstliche bzw. chemisch modifizierte Bestandteile zurück, die andererseits für das Erreichen einer zufriedenstellenden Haftung, d.h. Adhäsion zwischen Prothesen und den betreffenden Flächen im Mundraum verwendet werden. So haben beispielsweise Tests des Anmelders mit Haftcremes, die lediglich aus Flohsamen (sowie Öl und Kokosfett) hergestellt werden, gezeigt, dass bei zwar gegebener Verträglichkeit die Adhäsion der Zahnprothesen ungenügend ist. Es erweist sich mithin als diffizile Aufgabe, Haftmittel mit hoher Adhäsion aus rein natürlichen Komponenten anzufertigen.

**Aufgabe der Erfindung** ist es deshalb, eine Haftcreme für eine Kieferprothese oder eine Zahnprothese mit Gaumenplatte zur Verfügung zu stellen, die sowohl eine für die Prothesen geeignete hohe und lang anhaltende Adhäsion bewirkt, als auch dabei gut verträglich, möglichst geschmacksneutral und unbedenklich für eine Langzeitanwendung ist.

Die der Erfindung zu Grunde liegende Aufgabe wird durch eine Haftcreme mit den Merkmalen des Anspruchs 1 gelöst. Weitere vorteilhafte Ausgestaltungen der Erfindung sind in den Unteransprüchen zu Anspruch 1 angegeben. Ein zur Haftcreme korrespondierendes Herstellungsverfahren für diese Haftcreme wird in den Verfahrensansprüchen 7 bis 9 angegeben. Die Verwendung eines korrespondierenden Pulvers zur Herstellung der Haftcreme wird in Anspruch 6 angegeben.

Erfindungswesentlich ist die Verwendung von Stärke aus **Flohsamen** (Plantago ovata) und **Leinsamen** (Linum usitatissimum) und optional auch aus **Chia-Samen** (Salvia hispanica) als Hauptbestandteil der erfindungsgemäßen Haftcreme, wobei die Stärke einen Gewichtsprozentanteil von mehr als 50% der erfindungsgemäßen Haftcreme beträgt, wobei die verwendete Stärke durch Vermahlen und Abtrennen des Samens hergestellt wird. Da die Haftcreme neben der Stärke auch noch Öle und gegebenenfalls Fette aufweist, werden die besten Ergebnisse erzielt, wenn der Gehalt an Stärke aus Flohsamen und/oder Flohsamenschalen zwischen 50 Gewichtsprozent und 66 Gewichtsprozent bezogen auf die Gesamtmasse beträgt. Überraschender Weise hat sich gezeigt, dass die erfindungsgemäße Haftcreme, welche als wesentliches Füllmaterial Flohsamenstärke aufweist, einerseits ein gutes Haftvermögen zur Fixierung einer Kieferprothese oder einer Zahnprothese mit Gaumenplatte am menschlichen Gaumen aufweist. Andererseits ist der Geschmack der erfindungsgemäßen Haftcreme neutral bis gut verträglich, sofern geringfügige Mengen an Haftcreme während des Tragens in den Mundraum gelangen und damit verschluckt werden und schließlich in den Magen-Darm-Trakt gelangen. Schließlich ist die durch Verschlucken in den Magen-Darm-Trakt gelangende Haftcreme gut verdaulich und verträglich und dies auch für Menschen, die eine Intoleranz gegenüber modifizierten Stärken aufweisen. Intoleranz gegenüber modifizierten Stärken wird beispielsweise bei Menschen beobachtet, die eine Gluten-Intoleranz und/oder eine Lactose-Intoleranz zeigen und auch bei Menschen, die eine Malabsorption von verschiedenen Zuckern aufweisen. Bei diesen Menschen ist die normale Stärkeund Zuckerverdauung durch ein fehlendes Enzym gehemmt, so dass die üblicherweise in Haftcremes verwendeten Stärken nur bis zum Status einer Carbonsäure verdaut werden. Diese Carbonsäuren führen zu einem fauligen Stuhl, der Darm wird träge und die Verdauung endet in Diarrhoe oder Verstopfung.

Das überraschende Moment der Erfindung ist die Verwendung von Flohsamenstärke, Leinsamenstärke sowie optional auch Stärke aus Chia-Samen als Füllmittel für die erfindungsgemäße Haftcreme. Diese an sich gut verdauliche Stärke verleiht einerseits einer damit gefüllten erfindungsgemäßen Haftcreme im Mund eine akzeptable bis sehr gute Haftkraft und andererseits wirken Flohsamen im Darm als natürliches Darmregulans, wobei Flohsamen oder Flohsamenschalen aufgrund ihrer hohen Quellfähigkeit auch als pflanzliches Quellmittel oder Stuhlaufweicher verabreicht werden. Die löslichen Ballaststoffe reinigen zudem den Darm von Fäulnisstoffen und Darmgasen und wirken auch im Dünndarm. Sie fördern auch das Wachstum darmfreundlicher Bakterien. Da Flohsamen und/oder die Flohsamenschalen Schleimstoffe enthalten, die sich positiv auf die Gleitfähigkeit des Darminhaltes auswirken, können sich auch entzündliche Prozesse im Magen und Darm schneller zurückbilden. Flohsamen und/oder Flohsamenschalen werden in der Remissionsphase bei chronisch entzündlichen Darmerkrankungen eingesetzt, da sie einen schützenden Effekt auf die Darmschleimhaut ausüben. Durch die sehr gute Verträglichkeit von Flohsamen und/oder Flohsamenschalen können diese über eine lange Zeit im Mund verwendet werden

Eine nach dem Gedanken der Erfindung hergestellte Haftcreme zeichnet sich aus durch eine genügende Haftkraft über 18 bis 24 Stunden, eine angenehme Elastizität, einen neutralen Geschmack, eine angenehme Konsistenz, eine zum Einsetzen gut geeignete Fließeigenschaft, angenehm empfundene Trageigenschaften und durch eine einfache Reinigungsmöglichkeit. Im Stand der Technik ist die Reinigungsfähigkeit der Haftcreme ein häufiges Problem. Insbesondere die im Stand der Technik in den Haftcremes befindlichen Oleogele, Vaselinen und Mineralöle widersetzen sich häufig einer Reinigung mit herkömmlichen Reinigungsmitteln, die auf für den Einsatz im oder am menschlichen Körper gedachten Detergentien basieren. Im einfachsten Fall kann die Reinigung und Entfernung von Resten der erfindungsgemäßen Haftcreme mit klarem Wasser, eventuell mittels Zahnbürste erfolgen. Chemische Reinigungsmittel oder Öl sind für die erfindungsgemäße Haftcreme nicht erforderlich.

In einer Ausgestaltung der Erfindung kommt für die Stärke aus Leinsamen (Linum usitatissimum) als weiterem Bestandteil der erfindungsgemäßen Haftcreme in Betracht, dass die Leinsamenstärke einen Anteil an der erfindungsgemäßen Haftcreme zwischen 5 Gewichtsprozent und 10 Gewichtsprozent bezogen auf die Gesamtmasse hat. Bei Verwendung von natürlichen Stärken und Ölen ist in Kauf zu nehmen, dass sich stets ein geringer Anteil der erfindungsgemäßen Haftcreme durch die natürlichen Verdauungsprozesse im Mund auflöst. Sofern dieser Schwund an Haftcreme bewusst in Kauf genommen wird, sollten die sich auflösenden oder die verdauten Stoffe eine im Körper akzeptable bis positive Wirkung aufweisen. Im Fall von Leinsamen sind wichtige Nebenbestandteile der Stärke mehrfach ungesättigte Omega-3-Fettsäuren (Alpha- Linolensäure) und Schleimstoffe, die sich wiederum positiv auf die Magen- Darmschleimhaut auswirken. Leinsamenstärke enthält des Weiteren Eiweiß, Linamarin, Lecithin, Stearine, Vitamine Vitamin B1, Vitamin B2, Vitamin B6 und Vitamin E, Nicotinsäure, Folsäure und Pantothensäure.

Schließlich kommt in Ausgestaltung der Erfindung als weiterer Bestandteil **Chia-Samen** (Salvia hispanica) in Betracht und zwar bevorzugt in einem Gewichtsanteil von 0 Gewichtsprozent bis 10 Gewichtsprozent bezogen auf die Gesamtmasse. Chia-Samen sind sehr ölig und beinhalten bis zu 38% ihres Gewichtes ChiaÖl. Chia-Samen beinhalten einen hohen Anteil an Omega-3-Fettsäuren, hochwertigen Proteinen, Vitaminen, Mineralien, Ballaststoffe und als Antioxidantien wirkende Stoffe.

In Ausgestaltung der Erfindung kommt als ein weiterer Bestandteil der Stärkesorten in der erfindungsgemäßen Haftcreme eine Stärke aus **Dinkel** (Triticum aestivum subsp. spelta) in Betracht und zwar in einem Gewichtsanteil von 0,5 Gewichtsprozent bis 2,5 Gewichtsprozent bezogen auf die Gesamtmasse. Dinkel ist eine Getreideart und verwandt mit dem heutigen Weizen, welcher bei einer vorliegenden Weizenunverträglichkeit (Gluten-Intoleranz) des Nutzers gesundheitlich verträglicher ist als Weizen.

In weiterer Ausgestaltung der Erfindung kommt als ein weiterer Bestandteil der Stärkesorten in der erfindungsgemäßen Haftcreme eine Stärke aus **Hafer** (Triticum aestivum subsp. spelta) in Betracht und zwar in einem Gewichtsanteil von 2,5 Gewichtsprozent bis 4,5 Gewichtsprozent bezogen auf die Gesamtmasse. Hafer in Form von Haferflocken findet auch Verwendung als Lebensmittel für Patienten mit chronisch entzündlichen Darmerkrankungen.

In noch weiterer Ausgestaltung der Erfindung kommt als ein weiterer Bestandteil der Stärkesorten in dem erfindungsgemäßen Haftmittel eine Stärke aus **Naturreis** (Oryza sativa) in Betracht und zwar in einem Gewichtsanteil von 0 Gewichtsprozent bis 2,5 Gewichtsprozent bezogen auf die Gesamtmasse. Die Stärke aus Naturreis enthält viele Hauptnährstoffe sowie einen hohen Anteil an Kalium, Kalzium, Magnesium, Phosphor, Kupfer, Mangan, Zink, Bor, Fluor, Vitamine Vitamin E, Vitamin B1, Vitamin B2, Vitamin B6, und Pantothensäure.

Neben den Inhaltsstoffen aus Stärke kommen auch Kräuter und Kräuterextrakte in Betracht, um die Langzeitverträglichkeit der Haftcreme auf der Schleimhaut des Gaumens zu verbessern.

Um die Langzeitverträglichkeit zu verbessern, kommt in Ausgestaltung der Erfindung der Inhaltsstoff der **Kamillenblüte** (Matricaria chamomilla) in Betracht und zwar in einem Gewichtsanteil von 0,5 Gewichtsprozent bis 1,5 Gewichtsprozent bezogen auf die Gesamtmasse. Die Wirkung der Inhaltsstoffe der Kamillenblüten sind Entzündungshemmung und bei Magen-Darmbeschwerden und eine Wirkung gegen Blähungen. Des Weiteren hat das in den Kamillenblüten enthaltene Bisabolol eine antibakterielle Wirkung, was bei mechanischen Reizungen oder Traumatisierung des Gaumens von Vorteil ist, wenn die Gaumenplatte der Zahnprothese oder der Kieferprothese den Gaumen beim Kauen mechanisch reizt und somit einen Entzündungsherd im Mund eröffnet.

Ein weiterer Inhaltstoff, der in Ausgestaltung der Erfindung der erfindungsgemäßen Haftcreme beigefügt werden kann, ist **Petersilie** (Petroselinum crispum) und zwar in einem Gewichtsanteil von 0,5 Gewichtsprozent bis 1,5 Gewichtsprozent bezogen auf die Gesamtmasse. Die Petersilie enthält ätherisches Öl und sie wirkt durch ihren hohen Vitamin C-Gehalt belebend. Sie wirkt harntreibend, ist dadurch förderlich für Blase und Niere, und Petersilie wirkt positiv auf die Verdauung, da sie krampflösend sowie schleimlösend ist.

Um der Haftcreme eine cremeartige Konsistenz zu geben, kann sie mit einem **Pflanzenöl** versetzt werden und zwar in einem Gewichtsanteil von 10,0 Gewichtsprozent bis 15,0 Gewichtsprozent bezogen auf die Gesamtmasse. Bevorzugt sind solche Öle, die einen hohen Anteil an ungesättigten Fettsäuren enthalten, damit sie, wenn sie in den Magen-Darm-Trakt gelangen, dort durch die Verdauungsenzyme im Magen- und Darmtrakt leicht verdaulich sind und nicht durch ihre eigene Unverdaulichkeit eine Magenübersäuerung erzeugen. Hierzu kommen Pflanzenöle in Betracht ausgewählt aus der Gruppe bestehend aus: Palmenöl, Kokosöl, Rapsöl mit geringem Erucasäuregehalt und Sonnenblumenöl. Zur Herstellung der erfindungsgemäßen Haftcreme sollten nicht raffinierte Öle benutzt werden, da die Rohware kalt gepresst wird und dadurch mehr sekundäre Pflanzenstoffe enthalten sind als in raffinierten Ölen.

In besonderer Ausgestaltung der Erfindung kommt als weiterer Bestandteil **Kokosfett** in Betracht und zwar in einem Gewichtsanteil von 0 Gewichtsprozent bis 10,0 Gewichtsprozent bezogen auf die Gesamtmasse, um die Fließfähigkeit der Haftcreme zu beeinflussen. Kokosfett besitzt einen hohen Anteil an gesättigten Fettsäuren und wird als Lebensmittel zum Braten und Backen verwendet. Es besteht in der Hauptsache aus Triglyceriden sowie Spuren von Calcium, Kalium, Natrium, Kupfer, Eisen, Phosphor, Aminosäuren, Vitamin E und Lactone. Bei einer steten Aufnahme durch Schlucken und durch Verdauung im Mund ist durch die gute Verdaulichkeit keine Gefahr einer übermäßigen Gewichtszunahme zu befürchten.

Um die Bestandteile in der Haftcreme zu binden, ist in weiterer Ausgestaltung der Erfindung vorgesehen, dass **Agar-Agar** in der Haftcreme vorhanden ist und zwar in einem Gewichtsanteil von 0 Gewichtsprozent bis 2,5 Gewichtsprozent bezogen auf die Gesamtmasse. Agar-Agar wird aus den Zellwänden einiger Rotalgen gewonnen und liegt als langkettiges Kohlenhydrat vor. Der Hauptbestandteil des Agar-Agars ist die sogenannte Agarose.

Um die Bestandteile in der Haftcreme zu stabilisieren, ist in weiterer Ausgestaltung der Erfindung vorgesehen, **Xanthan** als Nebenbestandteil der Haftcreme einzusetzen und zwar in einem Gewichtsanteil von 0 Gewichtsprozent bis 2,5 Gewichtsprozent bezogen auf die Gesamtmasse. Xanthan wird bei ungewollter Aufnahme nur sehr wenig verstoffwechselt, und Xanthan wird mit Hilfe von Bakterien der Gattung Xanthomonas aus zuckerhaltigen Substraten gewonnen.

Um die hydrophilen Bestandteile der Haftcreme mit den öligen und fettigen Bestandteilen der Haftcreme zu emulgieren, ist in noch weiterer Ausgestaltung der Erfindung vorgesehen, **Sojalecithin** als Nebenbestandteil als Emulgator und zur Verbesserung der Bindung und Fließeigenschaft der Haftcreme einzusetzen und zwar in einem Gewichtsanteil von 0 Gewichtsprozent bis 2,5 Gewichtsprozent bezogen auf die Gesamtmasse. Sojalecithin wird ausschließlich als Reinlecithin aus der Sojabohne verwendet. Reinlecithin besitzt eine hohe Phospholipidkonzentration, wobei die Phospholipide die emulgierenden Eigenschaften zur Verfügung stellen. Sojalecithin ist geschmacksneutral und kann gut dosiert werden.

Das zur erfindungsgemäßen, mit den beschriebenen Bestandteilen ausgestalteten Haftcreme korrespondierende, erfindungsgemäße Verfahren zur Herstellung einer Haftcreme für Kieferprothesen oder Zahnprothesen mit einer Gaumenplatte zeichnet sich durch die folgenden Schritte aus: Vermahlen einer ersten Mischung von Flohsamen und Leinsamen und optional Chia-Samen; thermisches Behandeln der ersten Mischung durch Erhitzen auf 120 - 140°C für 15 min und abschließende Abkühlung auf 20°C bis 25°C; Vermahlen einer zweiten Mischung von Dinkel, Hafer, getrockneter Petersilie und Kamillenblüten und optional mindestens einem Bestandteil gewählt aus: Naturreis, Agar-Agar, Xanthan und/oder Soja-Lecithin; thermisches Behandeln der zweiten Mischung durch Erhitzen auf 120 - 140°C für 15 min und abschließender Abkühlung auf 20°C bis 25°C; Mischen der ersten und der zweiten thermisch behandelten Mischung mit mindestens einem Pflanzenöl als erstem Bestandteil einer dritten Mischung, ausgesucht aus der Gruppe bestehend aus: Palmenöl, Kokosöl, Rapsöl mit geringem (0,5 - 1,5 %) Erucasäuregehalt und Sonnenblumenöl, sowie mit Kokosfett als weiterem, optionalen Bestandteil der dritten Mischung.

Die erfindungsgemäße Haftcreme kann hergestellt werden aus einem einfachen Haftpulver, welches zu 100% aus Flohsamenstärke, Leinsamenstärke und wahlweise auch Chia-Samenstärke besteht. Dieses Haftpulver, das aus zwei Stärkesorten oder aus einer Mischung der Stärken der drei Samentypen besteht, wird zur Anwendung mit einem verträglichen Pflanzenöl und gegebenenfalls mit Kokosfett vermischt.

Zur Verdeutlichung der weiteren Ausgestaltungen der Erfindung werden im Folgenden Rezepte zur Herstellung der erfindungsgemäßen Haftcreme vorgestellt.

### I. erste Ausgestaltung der erfindungsgemäßen Haftcreme

In einem ersten Verfahrensschritt wird eine erste Mischung aus Flohsamenschalen und Leinsamen mit folgendem Mischungsverhältnis hergestellt:

### 1. Bestandteile der ersten Mischung

| **Bestandteil** | **Menge** | **Anteil** |
|---|---|---|
| Flohsamenschalen | 100,0 g | 86,2% |
| Leinsamen | 16,0 g | 13,8% |
| **SUMME** | **116,0 g** | **100,0 %** |

| | | |
|---|---|---|
| (Prozentanteile auf eine Stelle gerundet) | | |

Diese erste Mischung wird zu einem feinen Pulver vermahlen und das feine Pulver wird einer thermischen Behandlung unterzogen. Bei der thermischen Behandlung verändert sich die Stärke aufgrund einer chemischen Verkleisterung und unter anderem dient die Erhitzung auch, aber nicht nur, zur Desinfektion. Bei der thermischen Behandlung wird das feine Pulver für 18 min. auf 135°C erhitzt und wieder auf 20 bis 23°C abgekühlt.

In einem zweiten, parallelen oder darauf folgenden Verfahrensschritt wird eine zweite Mischung aus Dinkel, Hafer in Form von Haferflocken, getrockneter Petersilie und getrockneten Kamillenblüten mit folgendem Mischungsverhältnis hergestellt.

### 2. Bestandteile der zweiten Mischung

| **Bestandteil** | **Menge** | **Anteil** |
|---|---|---|
| Dinkel | 3,0 g | 18,8% |
| Haferflocken | 8,0 g | 50,0% |
| Petersilie | 2,0 g | 12,5 % |
| Kamillenblüten | 3,0 g | 18,8% |
| **SUMME** | **16,0 g** | **100,0%** |

| | | |
|---|---|---|
| (Prozentanteile auf eine Stelle gerundet) | | |

Diese zweite Mischung wird ebenfalls zu einem feinen Pulver vermahlen und das feine Pulver wird einer thermischen Behandlung unterzogen. Bei der thermischen Behandlung verändert sich die Stärke in Gegenwart der getrockneten Petersilie und der Kamillenblüten aufgrund einer chemischen Verkleisterung und unter anderem dient die Erhitzung auch, aber nicht nur, zur Desinfektion. Bei der thermischen Behandlung wird das feine Pulver für 15 min. auf 125°C erhitzt und wieder auf 20 bis 23°C abgekühlt.

Die beiden Pulver, nämlich das der ersten Mischung und das der zweiten Mischung werden sodann mit 32,4 g Pflanzenöl, ausgesucht aus der Gruppe bestehend aus Palmenöl, Kokosöl, Rapsöl mit geringem Erucasäuregehalt, Sonnenblumenöl, vermengt und in einem Mischer so lange gerührt, bis eine gleichmäßige, homogene Mischung entsteht.

Um eine pastöse Masse zu erhalten, kann die Haftcreme anschließend noch in einer Salbenmühle solange geschert werden, bis die Konsistenz der Haftcreme eine sich durch weiteres Scheren in der Salbenmühle nicht mehr verändernde Eigenschaft zeigt.

Nach dem Mischen und gegebenenfalls nach dem Scheren in der Salbenmühle wird die Haftcreme noch mindestens 15 min. ruhen gelassen. Danach kann die Haftcreme in Tuben abgefüllt werden.

Die Haftcreme der ersten Ausgestaltung der Erfindung weist die folgenden Bestandteile auf:

### 3. Mischungsanteile der Haftcreme

| **Bestandteil** | **Menge** | **Anteil** |
|---|---|---|
| Mischung 1 | 116,0 g | 70,6% |
| Mischung 2 | 16,0 g | 9,7 % |
| Pflanzenöl | 32,4 g | 19,7% |
| **SUMME** | **164,4 g** | **100%** |

| | | |
|---|---|---|
| (Prozentanteile auf eine Stelle gerundet) | | |

### 4. Bestandteile der Haftcreme

| **Bestandteil** | **Menge** | **Anteil** |
|---|---|---|
| Flohsamenschalen | 100,0 g | 61,0% |
| Leinsamen | 16,0 g | 9,8 % |
| Dinkel | 3,0 g | 1,8 % |
| Haferflocken | 8,0 g | 4,9 % |
| Petersilie | 2,0 g | 1,2 % |
| Kamillenblüten | 3,0 g | 1,8 % |
| Pflanzenöl | 32,4 g | 19,8 % |
| **SUMME** | **164,4 g** | **100,0** % |

| | | |
|---|---|---|
| (Prozentanteile auf eine Stelle gerundet) | | |

### II. zweite Ausgestaltung der erfindungsgemäßen Haftcreme

In einem ersten Verfahrensschritt wird eine erste Mischung aus Flohsamenschalen und Leinsamen mit folgendem Mischungsverhältnis hergestellt:

### 1. Bestandteile der ersten Mischung

| **Bestandteil** | **Menge** | **Anteil** |
|---|---|---|
| Flohsamenschalen | 100,0 g | 75,8 % |
| Leinsamen | 16,0 g | 12,1 % |
| Chia-Samen | 16,0 g | 12,1 % |
| **SUMME** | **132,0 g** | **100,0 %** |

| | | |
|---|---|---|
| (Prozentanteile auf eine Stelle gerundet) | | |

Diese erste Mischung wird zu einem feinen Pulver vermahlen und das feine Pulver wird einer thermischen Behandlung unterzogen. Bei der thermischen Behandlung verändert sich die Stärke aufgrund einer chemischen Verkleisterung und unter anderem dient die Erhitzung auch, aber nicht nur, zur Desinfektion. Bei der thermischen Behandlung wird das feine Pulver für 15 min. auf 125°C erhitzt und wieder auf 20 bis 23°C abgekühlt.

In einem zweiten, parallelen oder darauf folgenden Verfahrensschritt wird eine zweite Mischung aus Dinkel, Hafer in Form von Haferflocken, getrockneter Petersilie und getrockneten Kamillenblüten mit folgendem Mischungsverhältnis hergestellt.

### 2. Bestandteile der zweiten Mischung

| **Bestandteil** | **Menge** | **Anteil** |
|---|---|---|
| Dinkel | 3,0 g | 10,7 % |
| Haferflocken | 8,0 g | 28,6 % |
| Petersilie | 2,0 g | 7,1 % |
| Kamillenblüten | 3,0 g | 10,7 % |
| Naturreis | 3,0 g | 10,7 % |
| Agar-Agar | 3,0 g | 10,7 % |
| Xanthan | 3,0 g | 10,7 % |
| Soja-Lecithin | 3,0 g | 10,7 % |
| **SUMME** | **28,0 g** | **100,0** % |

| | | |
|---|---|---|
| (Prozentanteile auf eine Stelle gerundet) | | |

Diese zweite Mischung wird ebenfalls zu einem feinen Pulver vermahlen und das feine Pulver wird einer thermischen Behandlung unterzogen. Bei der thermischen Behandlung verändert sich die Stärke in Gegenwart der getrockneten Petersilie und der Kamillenblüten aufgrund einer chemischen Verkleisterung und unter anderem dient die Erhitzung auch, aber nicht nur, zur Desinfektion. Bei der thermischen Behandlung wird das feine Pulver für 15 min. auf 125°C erhitzt und wieder auf 20 bis 23°C abgekühlt.

Die beiden Pulver, nämlich das der ersten Mischung und das der zweiten Mischung werden sodann mit einer dritten Mischung bestehend aus einem Pflanzenöl, ausgesucht aus der Gruppe bestehend aus: Palmenöl, Kokosöl, Rapsöl mit geringem Erucasäuregehalt, Sonnenblumenöl, und Kokosfett als zweiten Mischungsbestandteil, die wie folgt zusammengesetzt ist, vermengt und in einem Mischer so lange gerührt, bis eine gleichmäßige, homogene Mischung entsteht.

### 3. Bestandteile der dritten Mischung

| **Bestandteil** | **Menge** | **Anteil** |
|---|---|---|
| Pflanzenöl | 32,0 g | 80,0% |
| Kokosfett | 8,0 g | 20,0% |
| **SUMME** | **40,0 g** | **100,0 %** |

| | | |
|---|---|---|
| (Prozentanteile auf eine Stelle gerundet) | | |

Um eine pastöse Masse zu erhalten, kann die Haftcreme anschließend noch in einer Salbenmühle solange geschert werden, bis die Konsistenz der Haftcreme eine sich durch weiteres Scheren in der Salbenmühle nicht mehr verändernde Eigenschaft zeigt.

### 4. Mischungsanteile der Haftcreme

| **Bestandteil** | **Menge** | **Anteil** |
|---|---|---|
| Mischung 1 | 132,0 g | 66,0 % |
| Mischung 2 | 28,0 g | 14,0 % |
| Mischung 3 | 40,0 g | 20,0 % |
| **SUMME** | **200,0 g** | **100 %** |

| | | |
|---|---|---|
| (Prozentanteile auf eine Stelle gerundet) | | |

Die Haftcreme der zweiten Ausgestaltung der Erfindung weist die folgenden Bestandteile auf:

| **Bestandteil** | **Menge** | **Anteil** |
|---|---|---|
| Flohsamenschalen | 100,0 g | 50,0 % |
| Leinsamen | 16,0 g | 8,0 % |
| Chia-Samen | 16,0 g | 8,0 % |
| Dinkel | 3,0 g | 1,5 % |
| Haferflocken | 8,0 g | 1,5 % |
| Petersilie | 2,0 g | 1,0 % |
| Kamillenblüten | 3,0 g | 1,5 % |
| Naturreis | 3,0 g | 1,5 % |
| Agar-Agar | 3,0 g | 1,5 % |
| Xanthan | 3,0 g | 1,5 % |
| Soja-Lecithin | 3,0 g | 1,5 % |
| Pflanzenöl | 32,0 g | 16,0 % |
| Kokosfett | 16,0 g | 8,0 % |
| **SUMME** | **200,0 g** | **100,0 %** |

| | | |
|---|---|---|
| (Prozentanteile auf eine Stelle gerundet) | | |

Nach dem Mischen und gegebenenfalls nach dem Scheren in der Salbenmühle wird die Haftcreme noch mindestens 15 min. ruhen gelassen. Danach kann die Haftcreme in Tuben abgefüllt werden.

Zur Verwendung der Haftcreme werden etwa 2 g auf die obere Gaumenplatte der Kieferprothese oder der Zahnprothese mit Gaumenplatte aufgetragen und die Gaumenplatte wird im Mund an den Gaumen gepresst, wobei der Nutzer die Gaumenplatte mit leichtem Kaudruck anpressen kann, um Lufteinschlüsse aus dem Bereich zwischen Gaumenplatte und Gaumen zu pressen. Durch die Berührung mit Speichel entsteht innerhalb von 15 min. bis 30 min. der erwünschte Hafteffekt.

Die Mischungsverhältnisse der einzelnen Bestandteile können in vorbestimmten Grenzen variieren. Als allgemeine Zusammensetzung kommen folgende Konzentrationsgrenzen in Betracht:
**1. Konzentrationsgrenzen der Bestandteile der ersten Mischung für 200 g Haftcreme**

| **Bestandteil** | **Menge, Untergrenze** | **Menge, Obergrenze** | **Anteil an 200 g** |
|---|---|---|---|
| Flohsamenschalen | 100,0 g | 132,0 g | 50,0-66,0% |
| Leinsamen | 10,0 g | 20,0 g | 5,0 - 10,0 % |
| Chia-Samen | 0,0 g | 20,0 g | 0,0 - 10,0 % |
| **SUMME** | **110,0 g** | **187,0 g** | **55,0 - 86,0 %** |

| | | | |
|---|---|---|---|
| (Prozentanteile auf eine Stelle gerundet) | | | |

**2. Konzentrationsgrenzen der Bestandteile der zweiten Mischung für 200 g Haftcreme**

| **Bestandteil** | **Menge, Untergrenze** | **Menge, Obergrenze** | **Anteil an 200 g** |
|---|---|---|---|
| Dinkel | 1,0 g | 5,0 g | 0,5 - 2,5 % |
| Haterflocken | 5,0 g | 9,0 g | 2,5 - 4,5 % |
| Petersilie | 1,0 g | 3,0 g | 0,5 - 1,5% |
| Kamillenblüten | 1,0 g | 3,0 g | 0,5 - 1,5 % |
| Naturreis | 0,0 g | 5,0 g | 0,0 - 2,5 % |
| Agar-Agar | 0,0 g | 5,0 g | 0,0 - 2,5 % |
| Xanthan | 0,0 g | 5,0 g | 0,0 - 2,5 % |
| Soja-Lecithin | 0,0 g | 5,0 g | 0,0 - 2,5 % |
| **SUMME** | **8,0 g** | **41,0%** | **4,0 - 20,0 %** |

| | | | |
|---|---|---|---|
| (Prozentanteile auf eine Stelle gerundet) | | | |

**3. Konzentrationsgrenzen der Bestandteile der dritten Mischung für 200 g Haftcreme**

| **Bestandteil** | **Menge, Untergrenze** | **Menge, Obergrenze** | **Anteil an 200 g** |
|---|---|---|---|
| Pflanzenöl | 20,0 g | 30,0 g | 10,0-15,0% |
| Kokosfett | 0,0 g | 20,0 g | 0,0 - 10,0 % |
| **SUMME** | **30,0 g** | **60,0 g** | **10,0 - 25,0 %** |

| | | | |
|---|---|---|---|
| (Prozentanteile auf eine Stelle gerundet) | | | |

## Patentansprüche

1. Haftcreme zur Fixierung einer Gaumenplatte einer Zahnprothese am menschlichen Gaumen,
**dadurch gekennzeichnet, dass**
die Haftcreme
- Flohsamenstärke in einem Anteil von 50 bis 66 Gew.-% bezogen auf das Gesamtgewicht der Haftcreme und
- Leinsamenstärke und
- als optionalen Bestandteil Chia-Samenstärke
enthält.

2. Haftcreme nach Anspruch 1,
**dadurch gekennzeichnet, dass**
diese aus mehr als einer Mischungskomponente besteht, wovon
- eine erste Mischungskomponente aus Flohsamenstärke, Leinsamenstärke und optional aus Chia-Samenstärke besteht, und
- die erste Mischungskomponente 55,0 bis 86,0 Gew.-% bezogen auf das Gesamtgewicht der Haftcreme ausmacht.

3. Haftcreme nach Anspruch 2,
**dadurch gekennzeichnet, dass**
diese aus mehr als einer Mischungskomponente besteht, wovon
- eine zweite Mischungskomponente Dinkel, Hafer, getrockneter Petersilie und Kamillenblüten enthält, und
- die zweite Mischungskömponente 4,0 bis 20,0 Gew.-% bezogen auf das Gesamtgewicht der Haftcreme ausmacht.

4. Haftcreme nach Anspruch 3,
**dadurch gekennzeichnet, dass**
die zweite Mischungskomponente zusätzlich Naturreis und/oder Agar-Agar und/oder Xanthan und/oder Soja-Lecithin aufweist.

5. Haftcreme nach einem der Ansprüche 3 bis 4,
**dadurch gekennzeichnet, dass**
diese aus mehr als einer Mischungskomponente besteht, wovon
- eine dritte Mischungskomponente
- aus mindestens einem Pflanzenöl,
ausgesucht aus der Gruppe bestehend aus Palmenöl, Kokosöl, Rapsöl mit geringem (0,5 - 1,5 %) Erucasäuregehalt und Sonnenblumenöl, und
- aus Kokosfett als optionalem Bestandteil besteht, und
- die dritte Mischungskomponente 10,0 bis 25,0 Gew.-% bezogen auf das Gesamtgewicht der Haftcreme ausmacht.

6. Verwendung eines Pulvers bestehend aus
- Flohsamenstärke und
- Leinsamenstärke und
- als optionalem Bestandteil Chia-Samenstärke zur Herstellung einer Haftcreme nach einem der Ansprüche 1 bis 5.

7. Verfahren zur Herstellung einer Haftcreme für Kieferprothesen oder Zahnprothesen mit einer Gaumenplatte,
aufweisend die folgenden Schritte:
- Vermahlen einer ersten Mischung von Flohsamen und Leinsamen und optional Chia-Samen,
- thermisches Behandeln der ersten Mischung durch Erhitzen auf 120 - 140°C für 15 min und abschließende Abkühlung auf 20°C bis 25°C,
- Vermahlen einer zweiten Mischung von Dinkel, Hafer, getrockneter Petersilie und Kamillenblüten und optional mindestens einem Bestandteil gewählt aus: Naturreis, Agar-Agar, Xanthan und/oder Soja-Lecithin,
- thermisches Behandeln der zweiten Mischung durch Erhitzen auf 120 - 140°C für 15 min und abschließender Abkühlung auf 20°C bis 25°C,
- Mischen der ersten und der zweiten thermisch behandelten Mischung mit mindestens einem Pflanzenöl als erstem Bestandteil einer dritten Mischung, ausgesucht aus der Gruppe bestehend aus: Palmenöl, Kokosöl, Rapsöl mit geringem (0,5 - 1,5 %) Erucasäuregehalt und Sonnenblumenöl, sowie mit Kokosfett als weiterem, optionalen Bestandteil der dritten Mischung.

8. Verfahren nach Anspruch 7,
wobei zur Homogenisierung die gesamte Mischung aus erster, zweiter und dritter Mischung in einer Salbenmühle bis zu einer über die Zeit gleich bleibenden Konsistenz geschert wird.

9. Verfahren nach einem der Ansprüche 7 oder 8,
**gekennzeichnet durch**
Ruhenlassen der Mischung für mindestens 15 min. bevor die Mischung abgefüllt wird.

## Claims

1. An adhesive cream for affixing an upper denture plate of a dental prosthesis onto the human palate,
**characterized in that**
the adhesive cream contains psyllium seed husk starch in an amount of
- 50 to 66 % by weight, based on the total weight of the adhesive cream, and
- linseed starch, and
- chia seed starch as an optional ingredient.

2. The adhesive cream according to claim 1,
**characterized in that**
it consists of more than one component of the mixture, wherein
- a first component of the mixture consists of psyllium seed husk starch, linseed starch and optionally chia seed starch, and
- the first component of the mixture constitutes 55.0 % to 86.0 % by weight, based on the total weight of the adhesive cream.

3. The adhesive cream according to claim 2,
**characterized in that**
it consists of more than one component of the mixture, wherein
- a second component of the mixture contains spelt, oats, dried parsley and chamomile blossoms, and
- the second component of the mixture constitutes 4.0 % to 20.0 % by weight, based on the total weight of the adhesive cream.

4. The adhesive cream according to claim 3,
**characterized in that**
the second component of the mixture additionally contains natural rice and/or agar and/or xanthan and/or soy lecithin.

5. The adhesive cream according to any one of claims 3 to 4,
**characterized in that**
it consists of more than one component of the mixture, wherein
a third component of the mixture consists of
- at least one vegetable oil,
- selected from the group consisting of palm oil, coconut oil, rapeseed oil with a low (0.5 - 1.5 %) erucic acid content and sunflower oil and
- coconut oil as an optional ingredient, and
- the third component of the mixture constitutes 10.0 % to 25.0 % by weight, based on the total weight of the adhesive cream.

6. Use of a powder, consisting of
- psyllium seed husk starch and
- linseed starch and
- chia seed starch as an optional ingredient,
to produce an adhesive cream according to any one of claims 1 to 5.

7. A method for producing an adhesive cream for maxillary prostheses or dental prostheses with an upper denture plate,
comprising the following steps:
- milling a first mixture of psyllium seed husks and linseed and optionally chia seeds,
- thermal treatment of the first mixture by heating to 120 - 140 °C for 15 minutes and then cooling to 20 °C to 25 °C,
- milling a second mixture of spelt, oats, dried parsley and chamomile blossoms and optionally at least one ingredient selected from: natural rice, agar, xanthan and/or soy lecithin,
- treatment of the second mixture by heating to 120 - 140 °C for 15 minutes and then cooling to 20°C to 25 °C,
- mixing the first and second thermally treated mixtures with at least one vegetable oil as the first ingredient of a third mixture, selected from the group consisting of palm oil, coconut oil, rapeseed oil with a low (0.5 - 1.5 %) erucic acid content and sunflower oil as well as with coconut oil as an additional optional ingredient of the third mixture.

8. The method according to claim 7,
wherein for homogenization the entire mixture of the first, second and third mixtures is subjected to shearing in an ointment mill until reaching a consistency that remains uniform over time.

9. The method according to any one of claims 7 or 8,
**characterized by**
leaving the mixture to stand for at least 15 minutes before bottling/packaging the mixture.

## Revendications

1. Crème adhésive destinée à fixer une plaque palatine d'une prothèse dentaire sur le palais humain,
**caractérisée en ce que**
la crème adhésive contient
- de l'amidon de psyllium dans une part de 50 à 66 % en poids, en rapport au poids total de la crème adhésive et
- de l'amidon de graines de lin et
- en tant que composant optionnel, de l'amidon de graines de Chia.

2. Crème adhésive selon la revendication 1,
**caractérisée en ce que**
celle-ci est constituée de plus d'un composant de mélange, dont
- un premier composant de mélange est constitué d'amidon de psyllium, d'amidon de graines de lin et en option, d'amidon de graines de Chia et
- le premier composant de mélange correspond à de 55,0 à 86,0 % en poids, en rapport au poids total de la crème adhésive.

3. Crème adhésive selon la revendication 2,
**caractérisée en ce que**
celle-ci est constituée de plus d'un composant de mélange, dont
- un deuxième composant de mélange contient de l'épeautre, de l'avoine, du persil séché et des fleurs de camomille et
- le deuxième composant de mélange correspond à de 4,0 à 20,0 % en poids, en rapport au poids total de la crème adhésive.

4. Crème adhésive selon la revendication 3,
**caractérisée en ce que**
le deuxième composant de mélange comporte en supplément du riz complet et/ou de l'agar-agar et/ou du xanthane et/ou de la lécithine de soja.

5. Crème adhésive selon l'une quelconque des revendications 3 et 4, **caractérisée en ce que**
celle-ci est constituée de plus d'un composant de mélange, dont
- un troisième composant de mélange
- est constitué d'au moins une huile végétale, choisie dans le groupe constitué de l'huile de palme, de l'huile de coco, de l'huile de colza à faible teneur (0,5 - 1,5 %) d'acide érucique et de l'huile de tournesol et
- de la graisse de coco, en tant que composant optionnel et
- le troisième composant de mélange correspond à de 10,0 à 25,0 % en poids, en rapport au poids total de la crème adhésive.

6. Utilisation d'une poudre, constituée
- d'amidon de psyllium et
- d'amidon de graines de lin et
- en tant que composant optionnel, d'amidon de graines de Chia
pour la fabrication d'une crème adhésive selon l'une quelconque des revendications 1 à 5.

7. Procédé destiné à fabriquer une crème adhésive pour des prothèses maxillaires ou des prothèses dentaires avec une plaque palatine,
comportant les étapes suivantes :
- broyage d'un première mélange de psyllium et de graines de lin et en option, de graines de Chia,
- traitement thermique du premier mélange par échauffement à de 120 - 140 °C pendant 15 minutes et refroidissement consécutif à de 20 °C à 25 °C,
- broyage d'un deuxième mélange d'épeautre, d'avoine, de persil séché et de fleurs de camomille et en option d'au moins un composant choisi parmi : le riz complet, l'agar-agar, le xanthane et/ou la lécithine de soja,
- traitement thermique du deuxième mélange par échauffement à de 120 - 140 °C pendant 15 minutes et refroidissement consécutif à de 20 °C à 25 °C,
- mélange du premier et du deuxième mélange ayant subi un traitement thermique avec au moins une huile végétale, en tant que premier composant d'un troisième mélange, choisi dans le groupe constitué de l'huile de palme, de l'huile de coco, de l'huile de colza à faible teneur (0,5 - 1,5 %) d'acide érucique et de l'huile de tournesol et ainsi qu'avec de la graisse de coco, en tant que composant supplémentaire optionnel du troisième mélange.

8. Procédé selon la revendication 7,
lors duquel, pour l'homogénéisation, on cisaille l'ensemble du mélange, constitué du premier, du deuxième et du troisième mélange dans un moulin à pommade, jusqu'à une consistance constante dans le temps.

9. Procédé selon l'une quelconque des revendications 7 ou 8,
**caractérisé par**
la mise au repos du mélange pendant au moins 15 minutes, avant de transvaser le mélange.
